# EUROPÄISCHE PATENTANMELDUNG

(11) **EP 0 563 604 A1**
(43) Veröffentlichungstag der Anmeldung: **06.10.1993**
(21) Anmeldenummer: 93103413.6
(22) Anmeldetag: 03.03.1993
(51) Int. Cl.: A61B 3/103

(54) **Verfahren und Vorrichtung zur Bestimmung der Refraktion von Augen**

(30) Priorität: 31.03.1992 DE 4210485
(71) Anmelder: Chiron Adatomed Pharmazeutische und Medizintechnische Gesellschaft mbH, D-85609 Dornach (DE)
(72) Erfinder: Reiner, Josef, Prof. Dr., W-5000 Köln 51 (DE)
(74) Vertreter: Nöth, Heinz, Dipl.-Phys.

(57) **Zusammenfassung**

Ein Verfahren und eine Vorrichtung zur Bestimmung der Refraktion von Augen, wobei eine Testfigur auf dem Augenhintergrund mit Hilfe eines Beleuchtungsstrahles eines Refraktometers (2) abgebildet wird und bei Scharfeinstellung der Testfigur die Augenrefraktion bestimmt wird. Hierfür kommt eine Zusatzeinrichtung zur Anwendung, bei welcher der Beleuchtungsstrahl, welcher insbesondere durch Infrarotlicht gebildet wird, durch zwei Fokussierlinsen (L1,L2), deren Brennpunkte zusammenfallen, geschickt wird und anschließend mit Hilfe eines teildurchlässigen Spiegels (3) auf das zu untersuchende Auge abgelenkt wird.

## Beschreibung

Die Erfindung betrifft ein Verfahren nach dem Oberbegriff des Patentanspruches 1 und eine Vorrichtung nach dem Oberbegriff des Patentanspruches 6 sowie eine Verwendung hierfür.

Es ist bekannt, zur Bestimmung der Augenrefraktion ein Augenrefraktometer zu verwenden. Dabei wird mit Hilfe eines Beleuchtungsstrahlenganges unter Mitwirkung des Augensystems eine Testfigur auf dem Augenhintergrund abgebildet. Durch verstellbare Elemente des Beleuchtungssystems wird dabei die Fehlsichtigkeit des Patienten ausgeglichen, so daß ein scharfes Bild der Testfigur auf dem Augenhintergrund entsteht. Bei astigmatischem Auge werden Teile der Testfigur scharf abgebildet, wobei eine Orientierung im Bereich zwischen 0° und 180° sich ergibt. Der Beleuchtungsausgang besitzt eine Austrittspupille, die außerhalb des Gerätes in die Pupille des zu prüfenden Auges zur Abbildung gelangt. Mittels mechanischer Einrichtungen ist die Einstellung des Refraktometers gegenüber dem Patientenauge möglich.

Mit Hilfe eines Beobachtungsstrahlenganges wird die Testfigur auf dem Augenhintergrund beobachtet. Der Beobachtungsstrahlengang besitzt eine Eintrittspupille, welche in der gleichen Ebene liegt wie die Austrittspupille des Beleuchtungssystems. Wenn diese beiden Pupillen in der gleichen Ebene liegen, ist das Refraktometer reflexfrei. Es sind zahlreiche Verfahren bekannt zur geometrischen Trennung dieser beiden Pupillen, um eine reflexfreie Beobachtung zu erreichen.

Bei klassischen manuellen Augenrefraktometern erfolgt die Einstellung des Testmarkenbildes auf dem Augenhintergrund des Patienten manuell während der Beobachtung mittels der Beobachtungsoptik. Zur Beobachtung wird Sichtbares Licht verwendet, das zur Blendung des Patienten führen und das Meßergebnis in Frage stellen kann.

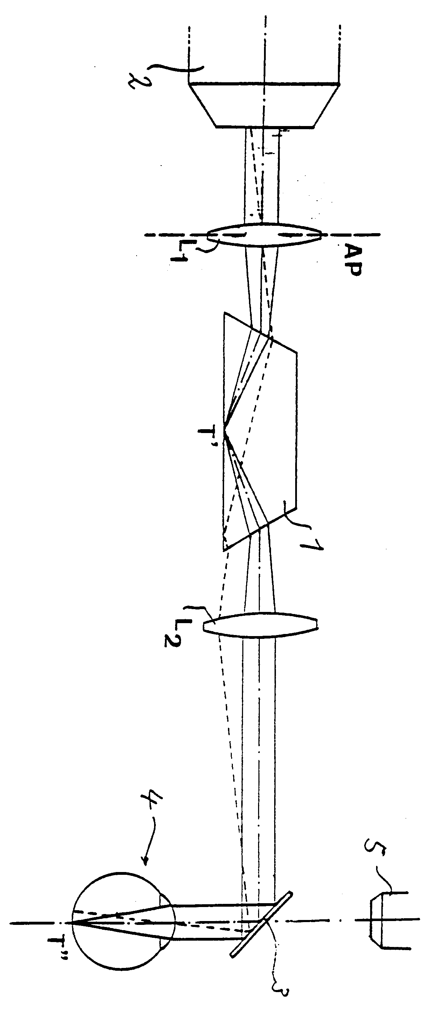

Bei automatischen Augenrefraktometern wird infrarote Strahlung verwendet. Die Abbildung der Testfigur auf dem Augenhintergrund wird vom Patientenauge nicht wahrgenommen. Es findet daher keine Blendung statt. Die Erfassung der abgebildeten Testfigur im Hinblick auf Scharfeinstellung erfolgt mit Hilfe von Sensoren, die eine selbsttätige Korrektur der Scharfeinstellung der Testfigur sowie eine Auswertung zur Augenrefraktionsbestimmung veranlassen (Z.prakt.Augenheilkd. 12:495-502,1991).

Die bekannten Augenrefraktometer werden so verwendet, daß der Patient in aufrechter Haltung vor dem Gerät sitzt. Die optische Achse des Patientenauges verläuft waagerecht, und zwar senkrecht zur Frontalebene. Der Kopf des Patienten wird durch geeignete Kopf- und Stirnstützen fixiert. Um eine Achseneinstellung bei astigmatischem Auge zu gewährleisten, können die Geräte mit Wasserwaagen oder anderen Horizontiervorrichtungen ausgestattet sein.

Aufgabe der Erfindung ist es, ein Verfahren und eine Vorrichtung der eingang genannten Art zu schaffen, bei denen der zu untersuchende Patent gegenüber dem Augenrefraktometer auch andere Positionen einnehmen kann.

Diese Aufgabe wird beim Verfahren der eingangs genannten Art erfindungsgemäß dadurch gelöst, daß zur Abbildung der Testfigur der Beleuchtungsstrahl durch zwei Fokussierlinsen, deren Brennpunkte zusammenfallen, geleitet wird und dann auf das zu untersuchende Auge abgelenkt wird.

Bei der eingangs genannten Vorrichtung wird diese Aufgabe erfindungsgemäß dadurch gelöst, daß die Mittel zum Abbilden der Testfigur im abbildenden Strahlengang zwei abbildende sammelnde Elemente, deren Brennpunkte zusammenfallen, aufweisen und daß zwischen dem Untersuchungsort und der Anordnung der beiden abbildenden sammelnden Elemente ein teildurchlässiger Ablenkspiegel angeordnet ist, der den abbildenden Beleuchtungsstrahlengang auf den Untersuchungsort richtet.

Hierdurch ist es möglich, daß der Patient gegenüber dem Refraktometer eine entfernt liegende Position einnimmt. Durch entsprechende Anordnung des Ablenkspiegels bzw. Ablenkung des Beleuchtungsstrahlenganges zum Untersuchungsort hin kann der Patient beispielsweise auch eine liegende Stellung - wie sie bei einer Augenoperation erforderlich ist - einnehmen. Vor allem können herkömmliche Augenrefraktometer unter Zuhilfenahme eines gemäß der Erfindung ausgebildeten Zusatzgerätes zur gleichzeitigen Refraktionsmessung während der Augenoperation verwendet werden.

Das Zusatzgerät, welches zwischen einem herkömmlichen Refraktometer und dem Behandlungsort, an welchem sich das zu untersuchende Auge, beispielsweise während, einer Augenoperation, befindet, enthält zwei abbildende sammelnde Elemente, deren Brennpunkte zusammenfallen. Die beiden Optikelemente können die gleiche Brennweite oder auch unterschiedliche Brennweiten aufweisen. Aufgrund dieses Zusatzgerätes ist es möglich, das Refraktometer aus einer beliebigen Richtung seitlich an das Patientenauge heranzuführen. Bevorzugt wird ein automatisches Refraktometer verwendet. Die beiden abbildenden sammelnden Optikelemente können durch Fokussierlinsen, entsprechend angeordnete Konkavspiegel und andere gleichwirkende optische Elemente dargestellt werden.

Um bei astigmatischem Auge die richtige Achse ermitteln zu können, kann die vom Beleuchtungsstrahlengang erzeugte Testfigur auf dem Augenhintergrund durch Drehung richtig orientiert werden. Diese Drehung kann mit Hilfe der im Refraktometer enthaltenen Auswerteeinrichtung, welche als Rechner ausgebildet ist, berücksichtigt bzw. durchgeführt werden. Es ist jedoch auch möglich, eine zusätzliche optische Dreheinrichtung, insbesondere in Form eines Drehprismas, vorzusehen, die bevorzugt zwischen den beiden Fokussierlinsen oder ähnlichen abbildenden sammelnden Optikelementen angeordnet ist.

Wenn man für die beiden abbildenden sammelnden Elemente die gleiche Brennweite wählt, so bleibt die Dioptrienanzeige des Refraktometers unverändert. Bei Veränderung des Brennweitenverhältnisses ändert sich die Vergrößerung und damit die Anzeige des Refraktometers.

Mit-Hilfe der Zusatzeinrichtung ist die Bestimmung der Scheitelbrechwerte des Auges nach Entfernen der trüben natürlichen Linse bei einer Katarakt-Operation möglich. Eine Überwachung der Scheitelbrechwerte bzw. der Augenrefraktionen und deren entsprechende Korrektur ist auch während der Implantation möglich, insbesondere, wenn die Implantation durch Injektion eines künstlichen Linsenersatzmaterials in den leeren Kapselsack (DE 40 11 053.2) durchgeführt wird, läßt sich diese Kontrolle bzw. Korrektur der Augenrefraktion vornehmen. Nach der Implantation kann eine Kontrolle der Augenrefraktion erfolgen. Eine Astigmatismus-Bestimmung aufgrund der Drehung der Testfigur, die - wie schon erläutert - rechnergestützt oder durch Prismendrehung oder Drehung anderer ungeradzahlig reflektierender Einrichtungen erfolgen kann, läßt sich ebenfalls erreichen.

Anhand der Figur, welche schematisch ein Ausführungsbeispiel der Erfindung darstellt, wird die Erfindung noch näher erläutert.

Für die Bestimmung der Refraktion an einem zu untersuchenden Auge, welches beispielsweise bei einer Augenoperation an einem Untersuchungsort 4 im Vorrichtungsaufbau angeordnet ist, wird mit Hilfe eines herkömmlichen Refraktometers 2 durchgeführt. Das Refraktometer 2 ist bevorzugt ein automatisches Refraktometer mit bekanntem Aufbau (Z.prakt.Augenheilkd. 12:495-502,1991). Zwischen dem Refraktometer 2 und dem Untersuchungsort 4 befindet sich das Zusätzgerät mit zwei Fokussierlinsen L₁ und L₂, welche die abbildenden sammelnden Optikelemente der Anordnung bilden. Die beiden Linsen L₁ und L₂ sind so angeordnet, daß ihre einander zugewandten Brennpunkte an einer Stelle T' zusammenfallen. Die Brennweiten der beiden Linsen können gleich oder unterschiedlich sein. Die erste Linse L₁ ist so angeordnet, daß sie mit einer Austrittspupille AP des Beleuchtungssystems des Augenrefraktometers 2 zusammenfällt. Die Linse L₁ nimmt somit die Position des zu untersuchenden Auges ein. In Wirklichkeit befindet sich jedoch das zu untersuchende Auge entfernt von der Austrittspupille AP am Untersuchungsort 4. Beim dargestellten Ausführungsbeispiel liegt der Untersuchungsort 4 in der doppelten Brennweite der zweiten Linse L₂. Auf diese Weise erreicht man einen Abstand zwischen dem Untersuchungsort 4 und der Austrittspupille AP des Refraktometers 2, welcher der vierfachen Brennweite der Linse L₁ entspricht, wenn die Linsen L₁ und L₂ gleiche Brennweite haben.

Um den Beleuchtungsstrahlengang auf das zu untersuchende Auge am Untersuchungsort 4 abzulenken, wird ein entsprechend schräg gestellter Ablenkspiegel 3 im Beleuchtungs-Strahlengang angeordnet. In bevorzugter Weise handelt es sich bei dem Ablenkspiegel 3 um einen teildurchlässigen Spiegel. Auf diese Weise erreicht man, daß der Beleuchtungsstrahlengang auf eine Vielzahl von möglichen Behandlungsorten gerichtet werden kann. Während einer Augenoperation befindet sich der Patient in liegender Stellung. Für diesen Fall hat der Ablenkspiegel 3 gegenüber der Richtung des von den beiden Linsen L₁ und L₂ kommenden Beleuchtungsstrahlenganges eine Neigung von 45°, so daß eine Ablenkung um 90° erreicht wird. Dies ist beim dargestellten Ausführungsbeispiel der Fall. Der Operateur kann durch den teildurchlässigen Spiegel, welcher für sichtbares Licht durchlässig ist, hindurchsehen und das Auge während der Operation und der Refraktionsmessung beobachten. Diese Beobachtung kann durch ein Operationsmikroskop herkömmlicher Bauart erfolgen. Von diesem Operationsmikroskop ist ein Objektiv 5 über dem Ablenkspiegel 3 in der Figur dargestellt.

Der Beleuchtungsstrahlengang des automatischen Refraktometers 2 wird bevorzugt von Infrarotstrahlen gebildet. Der teildurchlässige Ablenkspiegel 3 ist so ausgebildet, daß er nur Infrarotstrahlen reflektiert und sichtbares Licht hindurchläßt. Auf diese Weise wird die oben beschriebene Ablenkung des Beleuchtungsstrahlenganges auf den Untersuchungsort 4 bei gleichzeitiger Beobachtungsmöglichkeit des Auges durch den für sichtbares Licht durchlässigen Ablenkspiegel 3 erreicht.

Beim dargestellten Ausführungsbeispiel befindet sich zwischen den beiden Fokussierlinsen L₁ und L₂ als optische Dreheinrichtung ein um die optische Achse drehbares Prisma 1. Im Drehprisma 1 wird das von der Linse L₁ kommende Licht ungeradzahlig reflektiert.

Beim dargestellten Ausführungsbeispiel ist eine Reflexion dargestellt. Bei dem im Ausführungsbeispiel verwendeten Prisma handelt es sich um Dove-Prisma, welches ein geradsichtiges Prisma ist, bei dem einund austretender Strahl gleiche Richtung (Prismenablenkung 0°) haben. Bei der Drehung des Drehprismas 1 um die optische Achse dreht sich die Testfigur, so daß eine richtige Orientierung der Testfigur auf dem Augenhintergrund möglich ist. In Abhängigkeit von dem. Drehwinkel läßt sich für die Astigmatismus-Bestimmung die Achsenlage ermitteln.

Das Drehprisma 1 hat somit die Funktion der ungeradzahligen Reflexion zwischen den beiden Linsen L₁ und L₂ sowie die Funktion des Drehens der Testfigur. Die, Drehung der Testfigur kann jedoch auch durch eine zusätzliche Einrichtung an anderer Stelle des Beleuchtungsstrahlenganges oder durch die Auswerteeinrichtung des automatischen Refraktometers 2 bewirkt werden.

Die Testfigur wird im Augenhintergrund (Netzhaut) an einer Stelle T'' erzeugt. Im automatischen Refraktometer 2 sind entsprechende Sensoren vorgesehen, die in bekannter Weise den Beobachtungs-Strahlengang auswerten und selbsttätig eine Scharfeinstellung des Testbildes veranlassen. Aus den erforderlichen Einstellparametern wird dann die Refraktionsbestimmung in bekannter Weise gewonnen.

## Patentansprüche

1. Verfahren zur Bestimmung der Refraktion von Augen, bei dem eine Testfigur auf dem Augenhintergrund mit Hilfe eines Beleuchtungsstrahlenganges abgebildet und bei Scharfeinstellung und/oder Koinzidenzeinstellung der Testfigur die Augenrefraktion bestimmt wird,
dadurch **gekennzeichnet,**
daß zur Abbildung der Testfigur der Beleuchtungsstrahlengang durch zwei abbildende sammelnde Elemente, deren Brennpunkte zusammenfallen, geleitet und dann auf das zu untersuchende Auge abgelenkt wird.

2. Verfahren nach Anspruch 1,
dadurch gekennzeichnet, daß die abgebildete Testfigur gedreht wird.

3. Verfahren nach Anspruch 1 oder 2,
dadurch gekennzeichnet, daß die Stelle, an welcher die beiden Brennpunkte der abbildenden sammelnden Elemente zusammenfallen, auf oder außerhalb der optischen Achse liegt und um die optische Achse des die beiden abbildenden sammelnden Elemente durchsetzenden Strahlenganges gedreht wird.

4. Verfahren nach Anspruch 1 oder 2,
dadurch gekennzeichnet, daß der Strahlengang zwischen den beiden abbildenden sammelnden Elementen in ungerader Anzahl reflektiert wird.

5. Verfahren nach einem der Ansprüche 1 bis 4,
dadurch gekennzeichnet, daß der in die Anordnung der beiden abbildenden sammelnden Elemente einfallende Beleuchtungsstrahl in gleicher Richtung austritt.

6. Vorrichtung zur Bestimmung der Refraktion von Augen mit Mitteln zum Abbilden und Erfassen einer Testfigur auf dem Hintergrund eines an einem Untersuchungsort befindlichen Auges, Einstellmitteln zum Scharfeinstellen und/oder Koinzidenzeinstellen des Testbildes und einer Auswerteeinrichtung zur Bestimmung der Refraktionswerte bei scharfeingestelltem Testbild,
dadurch **gekennzeichnet,**
daß die Mittel zum Abbilden der Testfigur im abbildenden Beleuchtungsstrahlengang zwei abbildende sammelnde Elemente (L₁, L₂), deren Brennpunkte zusammenfallen, aufweisen und daß zwischen dem Untersuchungsort (4) und der Anordnung der beiden abbildenden sammelnden Elemente (L₁, L₁) ein teildurchlässiger Ablenkspiegel (3) angeordnet ist, der den abbildenden Beleuchtungsstrahlengang auf den Untersuchungsort (4) richtet.

7. Vorrichtung nach Anspruch 6,
dadurch gekennzeichnet, daß zwischen beiden abbildenden sammelnden Elementen (L₁, L₂) eine Einrichtung (1) mit ungeradzahliger Reflexion des Beleuchtungsstrahlenganges vorgesehen ist.

8. Vorrichtung nach Anspruch 6 oder 7,
dadurch gekennzeichnet, daß ferner eine Dreheinrichtung zum Drehen der Testfigur vorgesehen ist.

9. Vorrichtung nach einem der Ansprüche 6 bis 8,
dadurch gekennzeichnet, daß die Dreheinrichtung zum Drehen der Testfigur zwischen den beiden abbildenden sammelnden Elementen (L₁, L₂) angeordnet ist.

10. Vorrichtung nach einem der Ansprüche, 6 bis 9,
dadurch gekennzeichnet, daß die Dreheinrichtung als optische Einrichtung (1) mit ungeradzahliger Reflexion ausgebildet ist.

11. Vorrichtung nach Anspruch 10,
dadurch gekennzeichnet,daß die optische Einrichtung (1) als geradsichtiges Prisma ausgebildet ist.

12. Vorrichtung nach einem der Ansprüche 6 bis 11,
dadurch gekennzeichnet, daß die erste (L₁) der beiden abbildenden sammelnden Elemente (L₁, L₂) in einer Austrittspupille (AP) des Beleuchtungsstrahlenganges für die Abbildung der Testfigur angeordnet ist.

13. Vorrichtung nach einem der Ansprüche 6 bis 12,
dadurch gekennzeichnet, daß die beiden abbildenden sammelnden Elemente (L₁, L₂) gleiche Brennweite haben.

14. Vorrichtung nach einem der Ansprüche 6 bis 11,
dadurch gekennzeichnet, daß die beiden abbildenden sammelnden Elemente (L₁, L₂) ungleiche Brennweiten haben.

15. Vorrichtung nach einem der Ansprüche 6 bis 14,
dadurch gekennzeichnet, daß das die Testfigur bildende Licht Infrarotstrahlung ist.

16. Vorrichtung nach einem der Ansprüche 6 bis 15,
dadurch gekennzeichnet, daß der teildurchlässige Spiegel (3) für sichtbares Licht durchlässig ist und die die Testfigur bildende Strahlung reflektiert.

17. Vorrichtung nach einem der Ansprüche 6 bis 16,
dadurch gekennzeichnet, daß ein Mikroskop-Objektiv (5) durch den teildurchlässigen Spiegel (3) auf den Untersuchungsort (4) gerichtet ist.

18. Vorrichtung nach einem der Ansprüche 1 bis 16,
dadurch gekennzeichnet, daß die abbildenden sammelndenden Elemente (L₁, L₂) als Fokussierlinsen ausgebildet sind.

19. Verwendung einer Vorrichtung nach einem der Ansprüche 6 bis 18 bei einer Augenoperation zur gleichzeitigen Bestimmung der Augenrefraktion.
